(19) Europäisches Patentamt
European Patent Office
Office européen des brevets

(11) Publication number: **0 016 746**
**A1**

(12) # EUROPEAN PATENT APPLICATION

(21) Application number: **80850031.8**

(22) Date of filing: **17.03.80**

(51) Int. Cl.³: **C 07 C 87/459**
A 61 K 31/135, A 61 K 31/495
C 07 D 295/08

(30) Priority: **23.03.79 SE 7902647**

(43) Date of publication of application:
**01.10.80 Bulletin 80/20**

(84) Designated Contracting States:
**BE CH DE FR GB IT LU NL SE**

(71) Applicant: **Astra Läkemedel Aktiebolag**
**Strängnäsvägen 44**
**S-151 85 Södertälje(SE)**

(72) Inventor: **Carnmalm, Bernt Sigfrid Emanuel**
**Törnrosavägen 14**
**S-151 52 Södertälje(SE)**

(72) Inventor: **Rämsby, Sten Ingvar**
**Ägostigen 13**
**S-151 52 Södertälje(SE)**

(72) Inventor: **Stjernström, Nils Erik**
**Blomgatan 10C**
**S-752 31 Uppsala(SE)**

(72) Inventor: **Ögren, Sven Ove**
**Hökmossvägen 14B**
**S-155 00 Nykvarn(SE)**

(74) Representative: **Wurm, Bengt Runio et al,**
**Patent and Trade Mark Department Ab Astra**
**S-151 85 Södertälje(SE)**

(54) **New therapeutically active spirocycloalkylamines and pharmaceutical preparations comprising such compounds.**

(57) Compounds of the general formula

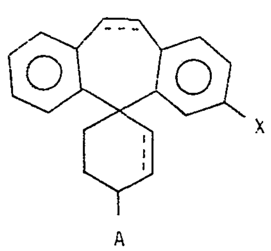

I

wherein the dotted lines represent optional double bonds, X represents a halogen or pseudohalogen selected from Cl, Br or $CF_3$, and A represents a monoloweralkylamino group or the group

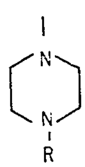

wherein R is a hydroxyloweralkyl group, pharmaceutical preparation and methods of treatment employing such compounds. The compounds are useful as neuropharmacological agents especially as neuroleptic or anti-psychotic agents.

EP 0 016 746 A1

# NEW THERAPEUTICALLY ACTIVE SPIROCYCLOALKYLAMINES AND PHARMACEUTICAL PREPARATIONS COMPRISING SUCH COMPOUNDS

## DESCRIPTION

### TECHNICAL FIELD

The present invention is related to new compounds of the spiro-dibenzocycloheptene-cyclohexaneamine type.

An object of the invention is to provide compounds for therapeutic use. A further object is to provide compounds having a therapeutic activity in the central nervous system.

### BACKGROUND ART

Among the compounds disclosed by US Patent 4 053 632 are compounds under the general formula

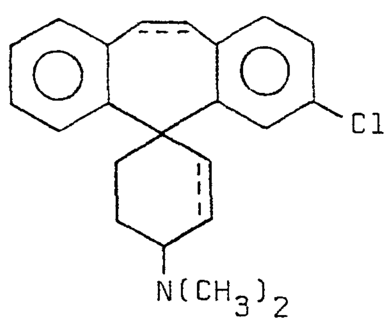

wherein the dotted lines represent optional double bonds. The compounds have neuroleptic properties.

### DISCLOSURE OF INVENTION

The present invention is related to compounds of the general formula

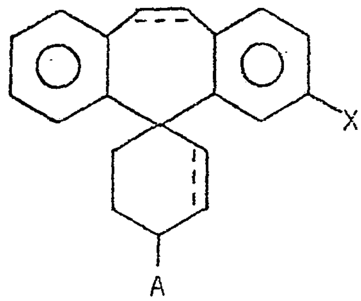

I

wherein the dotted lines represent optional double bonds, X represents a halogen or pseudohalogen selected from Cl, Br or $CF_3$ and A represents a monoloweralkylamino group or the group

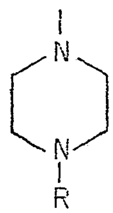

wherein R is a hydroxyloweralkyl group. Lower alkyl means an alkyl group containing 1 to 4 carbon atoms.

The hydroxy group, when occurring, is optionally esterified with a fatty acid having 3 to 20 carbon atoms. Pharmaceutically acceptable acid additions salts of the compounds of formula I are within the scope of the invention.

According to a preferred embodiment the invention is related to compounds of the formula

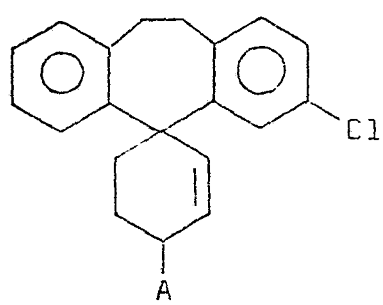

Ia

wherein A is as defined above.

Preferred compounds of the invention are those compounds of formula I or Ia in which A represents the group

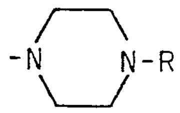

The compounds of the invention as defined above have valuable therapeutic properties as neuropharmacological agents, especially in use as neuroleptic or anti-psychotic agents. Further, the compounds of the invention have a reduced heart toxicity as compared with related known compounds. Additionally the compounds of formula I may be useful as anti-depressive or anxiolytic agents.

In a broadened concept the invention is related to compounds of formula I wherein A additionally represents the group $NH_2$. The compounds wherein A is $NH_2$ have a neuropharmacological activity and may be useful for treatment of certain neurological disorders such as depression. The primary amines are further useful as intermediates in preparation of other compounds of the invention.

The compounds of the invention exist in two diastereomeric forms, one wherein the chlorine atom and the nitrogen atom are in "cis" relationship to each other and one wherein said atoms are in "trans" relationship to each other. For convenience these diastereomers are referred to as "cis" and "trans" forms respectively.

In compounds of the invention containing a double bond in the six-membered hydrocarbon ring each diastereomer exists as two optical antipodes or enantiomers.

This invention also takes into consideration that compounds which structurally deviates from the formula I, after administration to a living organism may be transformed to a compound of the formula I and in this structural form exert

their effects. This consideration is a further aspect of this invention.

Methods of Preparation

The compounds of the invention may be obtained by one of the following methods, constituting a further aspect of the invention.

a) Reacting a compound of the formula

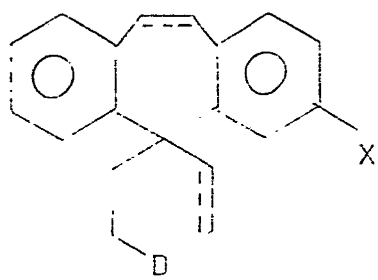

wherein D represents >C=O or 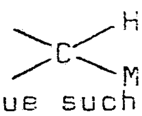 and M is a reactive acid residue such as a halogen atom, a lower alkylsulfonic or an arylsulfonic acid residue or such as a methylsulfonyl or tolylsulfonyl group, with an amine of the formula

H-A

wherein A is as defined above, whereby when D represents >C=O the reaction takes place in the presence of reducing agent. Thus, the process may follow a Leuckart-Wallach reaction carried out with heating in the presence of formic acid as the reducing agent, suitably after initially forming the formate of the amine. Preparation of starting compounds wherein D represents >C=O is described in J. Med. Chem 17 (1974) pages 65-72. Starting compounds wherein D represents 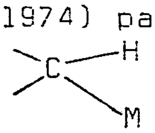 are obtainable by reducing the corresponding ketone in which D represents >C=O with a reducing agent, suitably a hydride reducing agent such as $LiAlH_4$ or $NaBH_4$, and esterifying the hydroxy group with an acid giving the residue M.

b) A compound of formula I wherein A represents a monoloweralkylamino group is also obtainable by alkylation of the corresponding primary amine by methods known per se. Thus the primary amine may be reacted with a haloformic acid ester and the carbamate formed be reduced to a compound of formula I wherein A is a methylamino, or the primary amine may be reacted with formamide and formic acid in an Eschweiler-Clarke reaction.

A compound defined by the formula I wherein A is replaced by $NH_2$ are preparable by method a) above using ammonia as the adduct and by the following additional methods:

Reduction of a compound of the formula

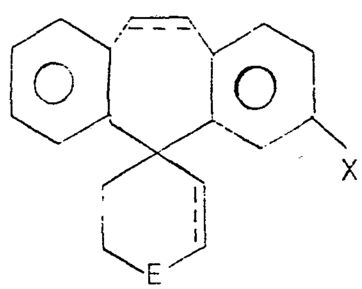

wherein E is >C= NOR', >C=NOCOR', >C=NOSO$_2$R', >C=NH, >C=NNHR', >CHN$_3$ or >CHNO$_2$ and R' is hydrogen, a lower alkyl group or an aryl group such as phenyl or tolyl.

Degrading a compound of the formula

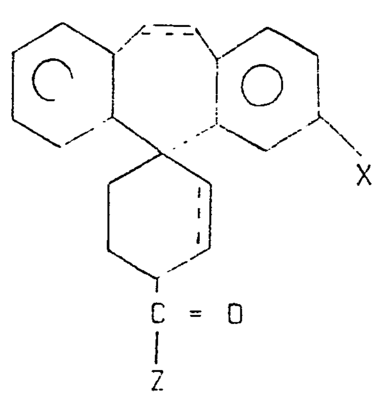

by one of the known rearrangement reactions to the formation of the primary amine of formula 1, whereby in a Schmidt reaction Z is a hydroxy group or an acid residue such as an organic acid residue of the formula R'COO- or an inorganic residue such as Cl, Br or I, in a Hofman reaction, Z is $NH_2$, in a Curtius reaction Z is $N_3$, and in a Lossen reaction Z is $-N^{\oplus}-O-CO-R'$, and R' is as defined above. The Schmidt reaction may be carried out under heating with hydrazoic acid or an inorganic salt thereof, suitably in the presence of a strong mineral acid. The Hofman reaction may be carried out by treatment of the amide with $Br_2$ or $Cl_2$ under alkaline conditions. The Curtius reaction may be carried out by heating the azide in an inert solvent and subsequent hydrolysis of the intermediate formed. The Lossen reaction may be carried out by heating the starting compoud in an aqueous solution, possibly under alkaline conditions, and, if necessary, hydrolysis of the compound formed.

Both organic and inorganic acids can be employed to form non-toxic pharmaceutically acceptable acid addition salts of the compounds of this invention.Illustrative acids are sulfuric, nitric, phosphoric, hydrochloric, citric, acetic, lactic, tartaric, pamoic, ethanedisulfonic, sulfamic, succinic, cyclohexylsulfamic, fumaric, maleic and benzoic acid. These salts are readily prepared by methods known in the art.

In clinical practice the compounds of the present invention will normally be administered orally, rectally, or by injection, in the form of pharmaceutical preparations comprising the active ingredient either as a free base or as a pharmaceutically acceptable non-toxic, acid addition salt, e.g. the hydrochloride, lactate, acetate, sulfamate, and the like, in association with a pharmaceutically acceptable carrier.

Accordingly, terms relating to the novel compounds of this invention, whether generically or specifically, are intended to include both the free amine base and the acid addition salts of the free base, unless the context in which such terms are used, e.g. in the specific examples, would be inconsistent with the broad concept. The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparation intended for injection and between 0.2 and 50% by weight for preparations suitable for oral administration.

Pharmaceutical preparations containing a compound of the invention in a solid form of dosage units for oral application may preferably contain between 2 and 50% by weight of the active substance, in such preparations the selected compound may be mixed with a solid fine grain carrier, e.g. lactose, saccharose, sorbitol, mannitol, starches such as potato starch, corn starch or amylopectin, cellulose derivatives, or gelatin and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatin, talcum, titanium dioxide, and the like. Alternatively the tablet can be coated with a lacquer dissolved in a readily volatile organic solvent or mixture of organic solvents. Dyestuffs may be added to these coatings in order to readily distinguish between tablets containing different active substances or different amounts of the active compound.

For the preparation of soft gelatin capsules (pearl-shaped) closed capsules)consisting of gelatin and, for example, glycerol or similar closed capsules, the active substance may be admixed with a vegetable oil. Hard gelatin capsules may contain granulates of the active substance in combination with solid, fine grain carriers such as lactose, saccharose, sorbitol, mannitol, starches (e.g. potato starch, corn starch or amylopectin), cellulose derivatives or gelatin.

Liquid preparations for oral application may be in the form of syrups or suspensions, for example, solutions containing from about 0.2% to about 20% by weight of the active substance herein described, the balance being sugar and a mixture of ethanol, water, glycerol and propyleneglycol. Optionally such liquid preparations may contain colouring agents, flavouring agents, saccharine and carboxymethylcellulose as a thickening agent.

Solutions for parenteral applications by injection can be prepared in an aqueous solution of a water-soluble pharmaceutically acceptable salt of the active substance preferably in a concentration of from about 0.5% to about 10% by weight. These solutions may also contain stabilizing agents and/or buffering agents and may conveniently be provided in various dosage unit ampoules.

In therapeutical treatment the suitable daily doses of the compounds of the invention are 5-500 mg for oral application, preferentially 50-250 mg and 1-100 mg for parenteral application, preferentially 10-50 mg.

The following examples will further illustrate the invention. In diastereomer ratios given the diastereomer with the shortest retention time is mentioned first.

Example 1

3-Chloro-10,11-dihydrospiro[5H-dibenzo[a,d]cycloheptene-5,1'-cyclohex-2'-en]-4'-one oxime

A solution of 3-chloro-10,11-dihydrospiro[5H-dibenzo[a,d]cycloheptene-5, 1'-cyclohex-2'-en]-4'-one (10.8 g, 35 mmol) and $NH_2OH \cdot HCl$ (10.0 g, 145 mmol) in 100 ml of pyridine was heated under reflux for 4 hours. The solution was concentrated to about half the volume and poured onto ice-water. After stirring for 1 hour the white precipitate was filtered off and washed several times with cold water. The dried product was recrystallized from acetonitrile giving 7.9 g (70%) of the oxime m.p. 183-185°C.

Example 2

3-Chloro-10,11-dihydrospiro[5H-dibenzo[a,d]cyclohepten-5,1'-cyclohex-2'-en]-4'-amine

To a solution of the oxime prepared according to Example 1 (7.0 g, 21.6 mmol) in 100 ml of dry benzene a solution of 50 ml of $NaAlH_2(OCH_2CH_2OCH_3)_2$ 70 % in benzene was added dropwise with stirring at ambient temperature. After the evolution of $H_2$ had ceased the solution was heated under reflux for 4 hours. The excess of hydride was destroyed by adding a saturated $Na_2SO_4$ solution. A conventional amine work-up procedure yielded the amine as a viscuous oil (6.3 g). The acetate was recrystallized from isopropyl alcohol, yield 5.9 g (74 %) m.p. 181-182°C. HPLC analysis with iso-octane+ethanol+conc $NH_3$, 90+10+0.1, as eluant showed a ratio of diastereomers of 30:70.

Example 3

3-Chloro-10,11-dihydro-N-methylspiro-[5H-dibenzo[a,d]-cycloheptene-5,1'-cyclohex-2-en]-4'-amine

Ethyl-chloroformate (1.95 g, 18 mmol) was added dropwise with stirring to a mixture of the amine prepared according to Example 2 (4.0 g, 13 mmol) in 20 ml of $CHCl_3$ and 10 ml of a 2 M NaOH solution at $0^oC$. After the addition the mixture was stirred for 15 minutes and then the $CHCl_3$ layer was washed with water and dried ($MgSO_4$). Work-up gave the corresponding ethyl carbamate (4.3 g) and the crude product was used in the next step without further purification.

A solution of the ethyl carbamate (3.7 g) in 25 ml of dry ether was added dropwise with stirring to a suspension of $LiAlH_4$ (2.0 g, 52 mmol) in 50 ml of dry ether $Et_2O$ at room temperature and then heated under reflux for 4 hours. Work-up gave the desired N-methyl amine as an oil (2.6 g). The maleate was precipitated from an ether solution with an equivalent amount of maleic acid. Recrystallization of the maleate from ethanol + water 1+1 gave 2.9 g (51%) from the primary amine of the amine maleate m.p. $231^oC$ (dec). Analysis of the product with HPLC showed it to contain the diastereomers in proportion 20:80.

Example 4

4-{3-Chloro-10,11-dihydrospiro[5H-dibenzo[a,d]cycloheptene--5,1'-cyclohex-2'-en-4'-yl]}-1-piperazineethanol

The same starting ketone as used in Example 1 (4.6 g, 15 mmol) was added to N-hydroxyethylpiperazine formate (5.3 g, 30 mmol), prepared by dropwise addition of HCOOH (1.4 g, 30 mmol) to N-hydroxyethylpiperazine (30 mmol) at

$0^oC$, and the mixture was heated at $160^oC$ for 6 hours. After cooling, the reaction mixture was poured into ice-water and work-up with $CHCl_3$ gave the amine as an oil (4.1 g). Recrystallization of the maleate from EtOH yielded the amine maleate 4.7 g (48%) m.p. $191^oC$ (dec). The dia-stereomeric composition was found to be 70:30.

Example 5

Preparation of tablets

a) Each tablet contains:

| | |
|---|---|
| Compound of Example 4 (maleate) | 10 mg |
| Lactose | 60 " |
| Starch | 29 " |
| Magnesium stearate | 1 " |

The powders are mixed and directly compressed to tablets with a diameter of 6 mm.

b)

| | |
|---|---|
| Compound of Example 4 (maleate) | 50 mg |
| Aerosil® (silicium dioxide) | 20 " |
| Lactose | 100 " |
| Starch | 30 " |
| Magnesium stearate | 2 " |

The active principle is mixed with the Aerosil®. This mixture is added to the other powders. Tablets are compressed with a diameter of 10 mm.

Example 6

Preparation of capsules

a)

| | |
|---|---|
| Compound of Example 4 (base) | 20 mg |
| Peanut oil | 60 mg |

The solution is filled into soft gelatine capsules, each capsule containing 20 mg of the active principle.

b)

| | |
|---|---|
| Compound of Example 4 (base) | 10 mg |
| Polyoxyethylene sorbitane monoleat | 100 " |

The capsules are made as described above.

The active substance employed in Examples 5 and 6 may be replaced by other pharmaceutically acceptable acid addition salts according to the invention.

Pharmacology

1. Neuroleptic effects

Behavioural and biochemical studies suggest that neuroleptic drugs such as chlorpromazine and haloperidol block central dopamine (DA) receptors. The DA receptor blockade as measured by different *in vitro* and *in vivo* techniques correlate roughly with the antipsychotic efficacy. The blockade of the behavioural effects induced by the DA receptor agonist apomorphine is highly correlated to the antipsychotic effect of various neuroleptics. The ability of potential neuroleptics to block the apomorphine induced behaviours may thus be a valuable predictor of the anti-

psychotic efficacy in man.

Methods

Male Sprague-Dawley rats, weighing 250-300 g, were used. The rats were observed in perspex cages and the behaviour was scored 5, 20, 40 and 60 min. after apomorphine. The compounds were injected intraperitoneally 60 min. prior to apomorphine hydrochloride (1 mg/kg dissolved in saline), which was injected subcutaneously into the neck. This dose and form of administration was found to produce a very consistent behavioural response and very low variation in the response strength. Directly after the injection the animals were placed in the cages, one in each cage. Scoring of the stereotypies was performed by a modified version (Fuxe et al. in Rhinencephale Neurotransmetteurs et Psychoses, by G. Masson, Paris 1977, p. 253) of the system introduced by Costall and Naylor (Eur. J. Pharmacol. 24 8 (1973).

The number of animals, displaying hyperactivity caused by apomorphine, was scored. Each group consisted of 6-8 animals. Saline controls were always run simultaneously. The $ED_{50}$'s for stereotypies are the doses which reduce the strength of the stereotypies by 50% over the observation period of 60 min. The $Ed_{50}$'s for hyperactivity are the doses which reduce the number of animals showing hyperactivity by 50% over the observation period of 60 min. $ED_{50}$'s were calculated from log dose-curves from 4-6 levels with 6-8 animals per dose level. The test compounds were dissolved in saline or water.

Results

The results are summarized in Table 1. The secondary amine (Example 3) was active and showed a preferential inhibition of the apomorphine induced hyperactivity. The hydroxyethyl--piperazine compound (Example 4) was found to block preferentially the apomorphine induced locomotion. Said compound was as potent as clozapine to block apomorphine induced hyperactivity and was more potent than clozapine to block the stereotypies.

Table 1

| Example No. | 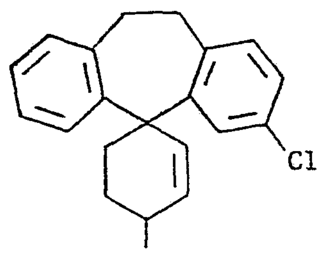<br>A | Diastereomer ratio | $ED_{50}$, µmol/kg i.p.<br>Stereo-typies | <br>Hyper-activity |
|---|---|---|---|---|
| | Compounds of the invention | | | |
| 3 | A = $-NHCH_3$ | 20:80 | 65 | 15 |
| 4 | 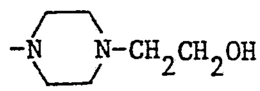 | 70:30 | 70 | 24 |
| | Prior art compounds | | | |
| | $-N(CH_3)_2$ | 55:45 | 35 | 6.4 |
| | Chlorpromazine | | 6.2 | 6.2 |
| | Clozapine | | 104 | 26 |

The apomorphine induced hyperactivity and stereotypies have been suggested to be due to stimulation of DA receptors in the limbic areas (e.g. nucleus accumbens) and striatum, respectively. It has also been proposed that the antipsychotic effect of the neuroleptics is connected with a blockade of DA receptors in the nucleus accumbens and the extrapyramidal side effects of these drugs to a blockade of DA receptors in the striatum. The results of the present study indicate that the amines of Examples 3 and 4 may have antipsychotic effects with low tendencies to induce extrapyramidal side effects.

## 2. Heart effects

### Methods

Male Sprauge-Dawley rats weighing 290-360 grams were used. The animals were prepared for recording the arterial blood pressure through a catheter implanted into the abdominal aorta and for injection of drugs through a catheter implanted into the abdominal vena cava. Both catheters were exteriorized at the base of the neck. At least five days were allowed for recovery after the operation.

The animals were kept immobilized in restraining cages during the experiment. The arterial blood pressure was continuously monitored on a Grass Model 7 Polygraph via a Statham P23Gc transducer. Electrocardiograms were taken from subcutaneously placed thin needle electrodes and recorded on a Mingograph 34 (Elema-Schönander AB, Sweden).

Groups of rats were given consecutive increasing intravenous doses of the test compounds from the dose scale: 0.25, 0.5, 1, 2, 4, 8, 16, 32 and 64 μmol/kg. The test compounds were injected over a period of 30 seconds at an interval of five minutes at doses of 0.25-8 μmol/kg and with an interval of 15 minutes at higher dose ranges.

The minimum doses that caused changes in mean arterial blood pressure, heart rate, PR, QRS and QT intervals of more than 15% from values before administration were arbitrarily defined as a drug effect.

### Results

The results are shown in Table 2. The compounds of the invention caused heart effects only in doses substantially higher than the dose of the prior art compound that caused such heart effects. While the prior art compound produced

arrhytmias at 2-4 μmol/kg the tested compounds of the invention produced such effects only after administration of 16 and 64 μmol/kg, respectively. It is concluded from these findings that the compounds of the invention are markedly less heart toxic than the prior art compound tested.

Table 2

Effects on intravenous administration of test compounds on arterial blood pressure, heart rate and electrocardiograms

| Example No. | 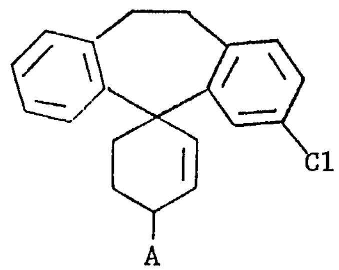 | Diastereomer ratio | Minimum doses (µmol/kg) causing changes in: Mean arterial blood pressure (Decrease) | Heart rate (Decrease) | Cardiogram intervals PR | QRS | QT | Minimum doses (µmol/kg) causing arrhythmias (Second degree AV-block) |
|---|---|---|---|---|---|---|---|---|
| Compounds of the invention | | | | | | | | |
| 3 | A = $-NHCH_3$ | 20:80 | 8-16 | 16 | 16 | 8 | 16 | 16 |
| 4 | A = 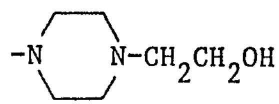 | 70:30 | 8 | 16-32 | 32 | 16 | 16 | 64 |
| Prior art compound | | | | | | | | |
| | A = $-N(CH_3)_2$ | 55:45 | 4 | 2 | 2 | >4 | 4 | 2-4 |

BEST MODE OF CARRYING OUT THE INVENTION

The compound 4-{3-chloro-10,11-dihydrospiro[5H-dibenzo-[a,d]cycloheptene-5,1'-cyclohex-2'-en-4'-yl]}-1-piperazine-ethanol, processes for preparing said compound and methods employing said compound represent the best mode of carrying out the invention known at present.

INDUSTRIAL APPLICABILITY

The invention is useful in the chemical and pharmaceutical industry and in health care.

CLAIMS

1. A compound characterized by the formula

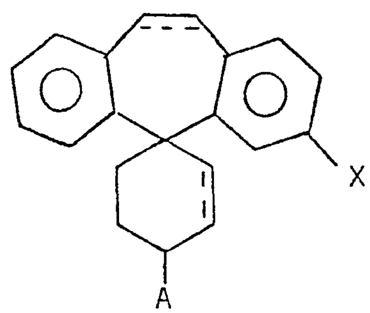

I

wherein the dotted lines represent optional double bonds, X represents a halogen or pseudohalogen selected from Cl, Br or $CF_3$ and A represents a monoloweralkylamino group or the group

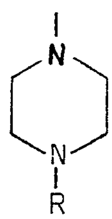

wherein R is a hydroxyloweralkyl group, wherein the hydroxy group when occurring is optionally esterified with a fatty acid having 3 to 20 carbon atoms, or a pharmaceutically acceptable acid addition salt thereof.

2. A compound according to claim 1 characterized by the formula

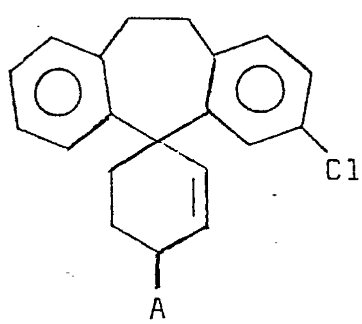

Ia

3. A compound according to claim 1 or 2, characterized in that A represents the group

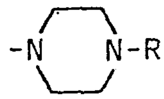 and R is as defined in claim 1.

4. A compound according to one or more of claims 1 to 3 in the form of a substantially pure cis or trans isomer.

5. A compound according to one or more of claims 1 to 4, in which the dotted line in the six membered hydrocarbon ring represents a double bond, in the form of a substantially pure enantiomer.

6. A compound characterized by the formula

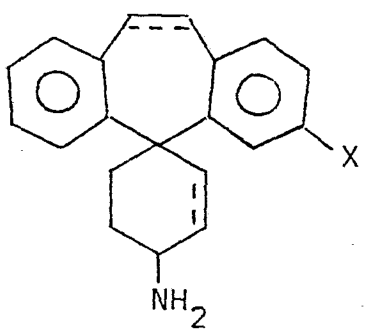

wherein the dotted lines represent optional double bonds, X represents a halogen or pseudohalogen selected from Cl, Br or $CF_3$, or a pharmaceutically acceptable acid addition salt thereof.

7. A pharmaceutical preparation comprising a therapeutically effective amount of a compound of the formula

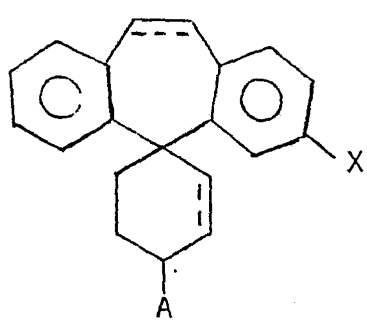

I

wherein the dotted lines represent optional double bonds, X represents a halogen or pseudohalogen selected from Cl, Br or $CF_3$ and A represents a monoloweralkylamino group or the group

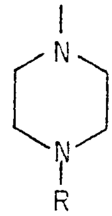

wherein R is a lower alkyl or hydroxyloweralkyl group wherein the hydroxy group when occurring is optionally esterified with a fatty acid having 3 to 20 carbon atoms, or a pharmaceutically acceptable acid addition salt thereof.

0016746

European Patent Office

# EUROPEAN SEARCH REPORT

Application number
EP 80850031.8

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| D | US - A - 4 053 632 (BERNT SIGFRID EMANUEL CARNMELM)<br>+ Claims 1-3,6,8 +<br>-- | 1,2,4-7 | C 07 C 87/459<br>A 61 K 31/135<br>A 61 K 31/495<br>C 07 D 295/08 |
| | US - A - 3 904 691 (BERNT SIGFRID EMANUEL CARNMALM)<br>+ Abstract +<br>-- | 1,2,4-7 | |
| | DE - A - 2 360 027 (ASTRA LÄKEMEDEL)<br>+ Claims 1,2 +<br>-- | 1,2,4,5 | TECHNICAL FIELDS SEARCHED (Int.Cl. 3) |
| | GB - A - 1 461 834 (AKZO N.V.)<br>+ Claims 1-6,12 +<br>---- | 1,7 | C 07 C 87/00<br>C 07 D 295/00 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |
| X | The present search report has been drawn up for all claims | | |

| Place of search | Date of completion of the search | Examiner |
|---|---|---|
| VIENNA | 12-06-1980 | REIF |